(19) Europäisches Patentamt
European Patent Office
Office européen des brevets

(11) Publication number: **0 164 463**
**B1**

(12) **EUROPEAN PATENT SPECIFICATION**

(45) Date of publication of patent specification: **11.05.88**

(21) Application number: **84303960.3**

(22) Date of filing: **12.06.84**

(51) Int. Cl.$^4$: **C 07 C 45/53,** C 07 C 49/403, C 07 C 49/39, C 07 C 49/395, C 07 C 49/413, C 07 C 45/33, C 07 C 179/053

# (54) Method for producing a mixture containing cycloalkanones and cycloalkanols.

(43) Date of publication of application:
**18.12.85 Bulletin 85/51**

(45) Publication of the grant of the patent:
**11.05.88 Bulletin 88/19**

(84) Designated Contracting States:
**DE GB NL**

(56) References cited:
**EP-A-0 027 937**
**US-A-4 341 907**

(73) Proprietor: **SUMITOMO CHEMICAL COMPANY, LIMITED**
**15 Kitahama 5-chome Higashi-ku**
**Osaka-shi Osaka 541 (JP)**

(72) Inventor: **Yashima, Akira**
**6-40, Maedacho**
**Niihama-shi Ehime 792 (JP)**
Inventor: **Matsuda, Teruo**
**8-6-73,Hoshigoecho**
**Niihama-shi Ehime 792 (JP)**
Inventor: **Sato, Tadao**
**3-8-19, Hachimancho**
**Niihama-shi Ehime 792 (JP)**
Inventor: **Sakai, Kiyomi**
**2-3-744, Ikkucho**
**Niihama-shi Ehime 792 (JP)**
Inventor: **Takahashi, Mitsuaki**
**6-22, Maedacho**
**Niihama-shi Ehime 792 (JP)**

(74) Representative: **Myerscough, Philip Boyd et al**
**J.A.Kemp & Co. 14, South Square Gray's Inn**
**London, WC1R 5EU (GB)**

**EP 0 164 463 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the Invention

This invention relates to a method for producing a mixture containing cycloalkanones and/or cycloalkanols which comprises oxidizing a cycloalkane with molecular oxygen to a cycloalkyl hydroperoxide and decomposing the cycloalkyl hydroperoxide.

The oxidation reaction of a cycloalkane with molecular oxygen generally belongs to an autoxidation reaction and comprises the stage where a cycloalkyl hydroperoxide is produced from a cycloalkane and molecular oxygen and the stage where said cycloalkyl hydroperoxide decomposes to produce a cycloalkanone and a cycloalkanol. The reaction of the first stage proceeds with relatively high yields while in the reaction of the second stage the decomposition degree of the cycloalkyl hydroperoxide differs greatly depending on the catalysts present in the system.

Hitherto, decomposition of cycloalkyl hydroperoxides has been effected in the presence of metallic salts. For example, "Kogyo Kagaku Zasshi" *73*, 2056 (1970)) reports the decomposition of cyclohexyl hydroperoxide with cobalt oxide, but this is not necessarily satisfactory because catalysts in high concentrations of several thousands ppm must be used to obtain sufficiently high decomposition rates.

Furthermore, "Kogyo Kagaku Zasshi", *73*, 2388 (1970) reports the decomposition of cyclohexyl hydroperoxide with cobalt naphthenate, etc. However, this requires the high decomposition temperature of 160°C in spite of using a high catalyst concentration of 10 ppm.

EP—A—48024 discloses the decomposition of cyclohexyl hydroperoxide with bis(pyridylimino)isoindoline complexes of cobalt and the like. However, according to this method the ratio of the resultant cyclohexanone and cyclohexanol is small, namely, about 0.2 and selectivity to cyclohexanone is low. Industrially, the mixtures of cyclohexanone and cyclohexanol are used mainly as starting materials for ε-caprolactam and adipic acid. Production of ε-caprolactam requires cyclohexanone and thus the step of dehydrogenation of cyclohexanol to cyclohexanone is necessary. Therefore, the higher production ratio of cyclohexanone is desired. When the mixture is used for production of adipic acid, the yield of adipic acid is maximum in the case of the ratio of cyclohexanone and cyclohexanol being 0.67 according to U.S. Patent No. 3,987,100. Thus, the higher production ratio of cyclohexanone is desired. That is, the higher production ratio is desired in decomposition of cyclohexyl hydroperoxide.

The inventors have made intensive researches on the method for producing a cycloalkanone and a cycloalkanol by decomposing a cycloalkyl hydroperoxide where decomposition rate of cycloalkyl hydroperoxide is high, yields of cycloalkanone and cycloalkanol are high, and the production ratio of cycloalkanone is high, and as a result this invention has been attained.

The present invention provides a method for producing a mixture containing cycloalkanone and/or cycloalkanol which comprises oxidizing a cycloalkane with molecular oxygen to a cycloalkyl hydroperoxide represented by the formula (1):

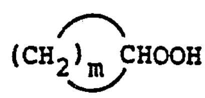 (1)

(wherein $4 \leq m \leq 11$) and decomposing the resultant cycloalkyl hydroperoxide to produce a mixture containing a cycloalkanone and/or a cycloalkanol, characterized by carrying out the oxidation of the cycloalkane with molecular oxygen and/or the decomposition of the cycloalkyl hydroperoxide in the presence of, as catalyst, either a mixture of a metallic salt of the formula (2):

$$MX_n \qquad (2)$$

(wherein M represents Co, Mn, Cr or Fe, X represents an anionic ligand and n represents 2 or 3) and an isoindoline of the formula (3):

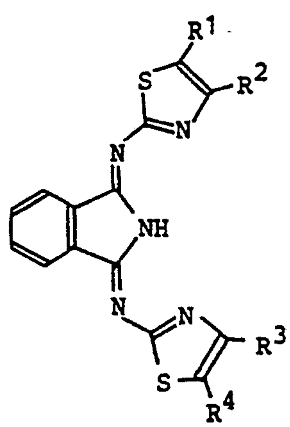 (3)

(wherein $R^1$, $R^2$, $R^3$ and $R^4$, which may be the same or different, each represent hydrogen or $C_1$—$C_6$ alkyl) or a metal complex represented by the formula (4):

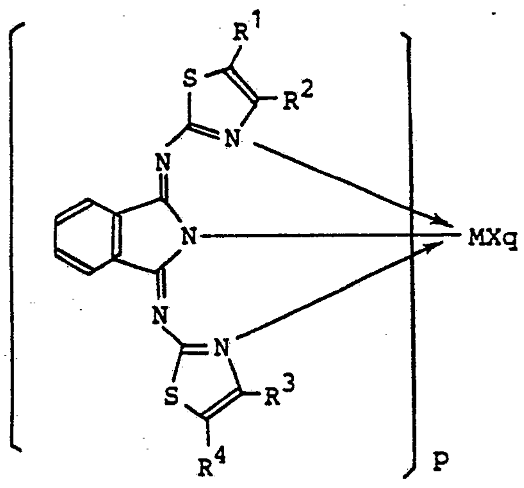

(4)

(wherein M represents Co, Mn, Cr or Fe, X represents an anionic ligand, $R^1$, $R^2$, $R^3$ and $R^4$, which may be the same or different, each represent hydrogen or a $C_1$—$C_6$ alkyl, p is 1 or 2 and q is 0, 1 or 2).

Advantages of the method of this invention over the conventional methods are, for example, that higher conversion of cycloalkyl hydroperoxide is attained than with cobalt naphthenate and higher yields of cycloalkanone and cycloalkanol are obtained than with cobalt naphthenate and that higher production ratio of cycloalkanone is obtained than with bis(pyridylimino)isoindoline complex of cobalt.

The cycloalkanes used in this invention are those which contain 5 to 12 carbon atoms such as cyclopentane, cyclohexane, cyclooctane, cyclododecane. Industrially, cyclohexane is especially preferred.

The cycloalkyl hydroperoxides (1) are those which are obtained by oxidation of cycloalkanes with molecular oxygen. As examples of them, mention may be made of cyclopentyl hydroperoxide, cyclohexyl hydroperoxide, cyclooctyl hydroperoxide, cyclododecyl hydroperoxide, etc. in correspondence to the above exemplified cycloalkanes.

With reference to the metallic compounds (2), the metal M is Co, Mn, Cr or Fe, and preferably Co.

The anionic ligands X are selected from organic acid groups, inorganic acid groups, hydroxyl groups and oxygen. Examples of the organic acids are carboxylic acids, organic sulfonic acids, organic phosphoric acids, etc. Examples of the carboxylic acids are acetic acid, propionic acid, 2-ethylhexanoic acid, octylic acid, naphthenic acids, etc. Examples of the organic sulfonic acids are p-toluenesulfonic acid, dodecylbenzenesulfonic acid, etc. Examples of the organic phosphoric acids are monotridecylphosphoric acid, etc. Examples of the inorganic acids are sulfuric acid, nitric acid, etc.

As examples of the metallic compounds (2), mention may be made of Co salts, Mn salts, Cr salts and Fe salts of acetic acid, 2-ethylhexanoic acid, naphthenic acid, p-toluenesulfonic acid, monotridecylphosphoric acid and sulfuric acid, hydroxides of Co, Mn, Cr and Fe and oxides of Co, Mn, Cr and Fe. Preferred are Co salts of carboxylic acids, especially preferred are cobalt 2-ethylhexanoate, cobalt octylate and cobalt naphthenate.

Isoindolines (3) include 1,3-bis(2-thiazolylimino)isoindoline (abbreviated as "BTIH" hereinafter) and derivatives thereof.

The lower alkyl groups of the isoindolines mean alkyl groups of 1 to 6 carbon atoms such as methyl, ethyl, hexyl, t-butyl, etc.

Examples of the isoindolines (3) are 1,3-bis(2-thiazolylimino)isoindoline, 1,3-bis(4-methyl-2-thiazolylimino)isoindoline (abbreviated as "4MeBTIH" hereinafter), 1,3-bis(5-methyl-2-thiazolylimino)isoindoline (abbreviated as "5MeBTIH" hereinafter), etc.

As mentioned above, the catalyst composition used in the method of this invention is a mixture of the metallic compound (2) and the isoindoline (3), but may also be the metal complex (4).

In the metal complexes (4), M, X, $R^1$, $R^2$, $R^3$ and $R^4$ are as mentioned hereinabove and examples thereof are $Co(BTI)_2$, $Co(4MeBTI)_2$, $Co(5MeBTI)_2$, $Co(BTI)OCOCH_3$, etc. which are cobalt derivatives of BTIH.

It can be presumed that the mixtures of the metallic salt compound (2) and the isoindoline (3) produce compounds similar to the metal complexes (4) in the reaction systems and thus the two catalyst compositions can be said to be essentially identical.

This invention can be applied to both the cases where oxidation of cycloalkanes and decomposition of cycloalkyl hydroperoxides are separately carried out and simultaneously carried out.

Decomposition of cycloalkyl hydroperoxides is usually effected in solutions in suitable solvents. Suitable solvents are alkanes such as hexane and octane, cycloalkanes such as cyclopentane and cyclohexane, aromatic hydrocarbons such as benzene, etc. Especially preferred are cycloalkanes corresponding to the cycloalkyl hydroperoxides.

These solutions may be those of isolated cycloalkyl hydroperoxides dissolved in the solvents, but cycloalkane oxidation reaction mixtures per se or those which are diluted or concentrated to a suitable concentration are preferred.

Concentration of the cycloalkyl hydroperoxides used in the method of this invention is 0.1 to 10 % by weight, preferably 0.5 to 5 % by weight.

Amount of the metallic compounds (2) (in terms of metal) used in the method of this invention is 0.1 to 100 ppm, preferably 0.3 to 5 ppm of the entire mixture. When less than 0.1 ppm, sufficient effect cannot be exhibited and when more than 100 ppm, the effects are no longer increased.

Amount of the isoindolines (3) is 0.1 to 100, preferably 1 to 10 in molar ratio to the metallic compound (2). When less than 0.1, the effects are small and when more than 100, the effects are no longer increased.

The metallic compound (2) and the isoindoline (3) may be added separately to the reaction mixture or they may be added as a mixture.

When the metal complex (4) is used, amount thereof (in terms of metal) is 0.1 to 100 ppm, preferably 0.3 to 5 ppm of the entire compound. When less than 0.1 ppm, the effects are small and when more than 100 ppm, no further increase of the effects can be expected.

The metallic compounds (2), the isoindolines (3) and the metal complex (4) used in the method of this invention may be added as they are, but they may also be added as solutions in solvents such as hydrocarbons, alcohols, ketones, etc.

The method of this invention may be carried out in the presence or absence of molecular oxygen. When molecular oxygen is present, yields of products cycloalkanones and cycloalkanols can be increased without disturbing the decomposition of cycloalkyl hydroperoxide. Thus, presence of molecular oxygen is preferred.

Oxygen is generally used as mixtures with inert gases such as argon and nitrogen. Examples of such mixtures are air, oxygen-added air and nitrogen-added air and air without any pretreatment may be advantageously used.

Reaction temperature employed in the method of this invention may be 80 to 200°C, especially preferably 100 to 160°C. Reaction time is 1 to 120 minutes and reaction pressure is 2 to 20 kg/cm$^2$G. (0,2 to 2 MPa gauge).

This invention will be further explained in the following Examples.

In these Examples, conversion, selectivity and production ratio were calculated by the following formulas.

$$\text{Conversion (\%)} = \frac{\text{Consumed cyclohexyl hydroperoxide}}{\text{Charged cyclohexyl hydroperoxide}} \times 100$$

$$\text{Selectivity (\%)} = \frac{\text{Produced cyclohexanone} + \text{Produced cyclohexanol}}{\text{Consumed cyclohexyl hydroperoxide}} \times 100$$

$$\text{Production ratio of cyclohexanone and cyclohexanol} = \frac{\text{Produced cyclohexanone}}{\text{Produced cyclohexanol}}$$

Example 1

In a glass autoclave were charged 0.5 part by weight of cyclohexyl hydroperoxide, 50 parts by weight of cyclohexane, 0.00005 part by weight (in terms of cobalt) of cobalt naphthenate and 0.0003 part by weight of BTIH. Then, gas phase part in the autoclave was replaced with nitrogen and thereafter decomposition reaction was carried out while stirring at 140°C for 20 minutes. After completion of the reaction the reaction mixture was taken out from the autoclave and this reaction was analyzed by gas chromatography and iodometry. The results were as follows: conversion of cyclohexyl hydroperoxide 99 %; selectivity to cyclohexanone and cyclohexanol 130 %; and production ratio of cyclohexanone and cyclohexanol 0.34.

Comparative Example 1

Example 1 was repeated except that BTIH was not added. The results were as follows: conversion of cyclohexyl hydroperoxide 40 %; selectivity to cyclohexanone and cyclohexanol 115 %; and production ratio of cyclohexanone and cyclohexanol 0.50.

Comparative Example 2

Example 1 was repeated except that 0.0003 part by weight of 1,3-bis(2-pyridylimino)isoindoline was used in place of BTIH. The results were as follows: conversion of cyclohexyl hydroperoxide 99 %; selectivity to cyclohexanone and cyclohexanol 128 %; and production ratio of cyclohexanone and cyclohexanol 0.23.

Example 2

In a glass autoclave of 500 ml internal volume were charged 0.0004 part by weight of Co(BTI)$OCOCH_3$ and 50 parts by weight of a mixture containing 0.5 part by weight of cyclohexyl hydroperoxide and totally 2 parts by weight of cyclohexanone and cyclohexanol, said mixture being obtained by liquid phase oxidation of cyclohexanone with air. Then, the autoclave was closed keeping air atmosphere in the gas phase part of the autoclave and subsequently decomposition reaction was carried out while stirring at 140°C for 60 minutes. After the reaction, the reaction mixture was taken out from the autoclave and analyzed in the same manner as in Example 1. The results were as follows: conversion of cyclohexyl hydroperoxide 99 %; and selectivity to cyclohexanone and cyclohexanol 220 %.

**Claims**

1. A method for producing a mixture containing a cycloalkanone and/or a cycloalkanol which comprises oxidizing a cycloalkane with molecular oxygen to a cycloalkyl hydroperoxide represented by the formula (1):

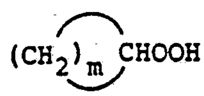

(1)

(wherein $4 \leqq m \leqq 11$) and decomposing the resultant cycloalkyl hydroperoxide to produce a mixture containing a cycloalkanone and/or a cycloalkanol, characterised by carrying out the oxidation of the cycloalkane with molecular oxygen and/or the decomposition of the cycloalkyl hydroperoxide in the presence of, as catalyst, either a mixture of a metallic compound of the formula (2):

$$MX_n \qquad (2)$$

(wherein M represents Co, Mn, Cr or Fe, X represents an anionic ligand and n is 2 or 3) and an isoindoline of the formula (3):

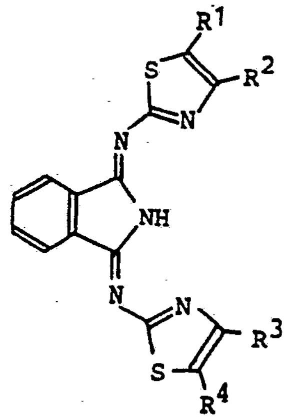

(3)

(wherein $R^1$, $R^2$, $R^3$ and $R^4$, which may be the same or different, each represent hydrogen or $C_1$—$C_6$ alkyl) or a metal complex represented by the formula (4):

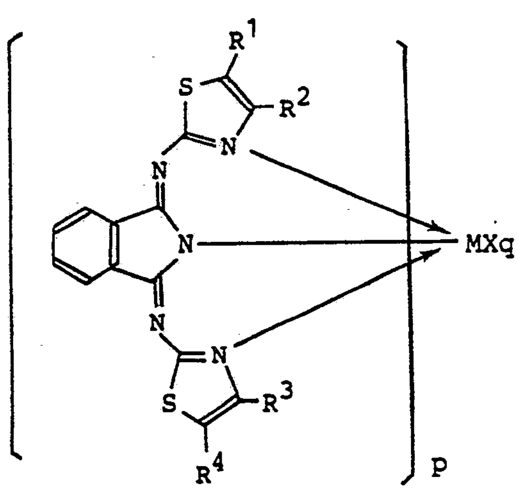

(4)

(wherein M represents Co, Mn, Cr or Fe, X represents an anionic ligand, $R^1$, $R^2$, $R^3$ and $R^4$, which may be the same or different, each represent hydrogen or $C_1$—$C_6$ alkyl, p is 1 or 2 and q is 0, 1 or 2).

2. A method according to claim 1 wherein the cycloalkane to be oxidized with molecular oxygen has 5 to 12 carbon atoms.

3. A method according to claim 1 or 2 wherein the metal M in the metallic compound (2) and the metal complex (4) is Co.

4. A method according to claim 1, 2 or 3 wherein the anionic ligand X in the metallic compound (2) and the metal complex (4) is an organic acid group, inorganic acid group or oxygen.

5. A method according to any one of the preceding claims wherein the concentration of the cycloalkyl hydroperoxide is 0.1 to 10 % by weight.

6. A method according to any one of the preceding claims wherein the molar ratio of the isoindoline (3) to the metallic compound (2) is 0.1/1 to 100/1.

7. A method according to any one of the preceding claims wherein the amount of the metallic compound (2) or metal complex (4) in terms of metal is 0.1 to 100 ppm of the entire mixture.

8. A method according to any one of the preceding claims wherein the reaction temperature is 80 to 200°C.

9. A method according to any one of the preceding claims wherein the reaction time is 1 to 120 minutes.

10. A method according to any one of the preceding claims wherein the reaction pressure is 2 to 20 kg/ $cm^2$G, (0,2 to 2 MPa gauge).

**Patentansprüche**

1. Verfahren zur Herstellung einer Mischung, die ein Cycloalkanon und/oder ein Cycloalkanol enthält, durch Oxidation eines Cycloalkans mit molekularem Sauerstoff zu einem Cycloalkylhydroperoxid der Formel (1):

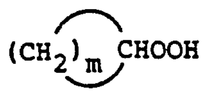

(1)

(worin 4 ≦ m ≦ 11) und Zersetzung des entstehenden Cycloalkylhydroperoxids, um eine Mischung herzustellen, die ein Cycloalkanon und/oder ein Cycloalkanol enthält, dadurch gekennzeichnet, daß die Oxidation des Cycloalkans mit molekularem Sauerstoff und/oder die Zersetzung des Cycloalkylhydroperoxids in Anwesenheit von entweder einem Gemisch einer Metallverbindung der Formel (2):

$$MX_n \qquad (2)$$

(worin M Co, Mn, Cr oder Fe bedeutet, X für einen anionischen Liganden steht und n den Wert 2 oder 3 hat) und einem Isoindolin der Formel (3):

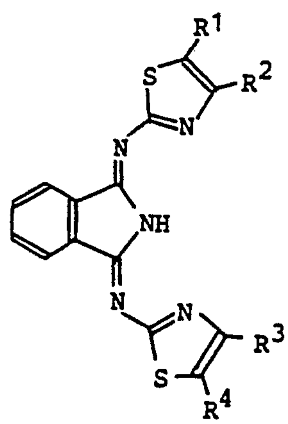

(3)

(worin $R^1$, $R^2$, $R^3$ und $R^4$, die gleich oder unterschiedlich sein können, je Wasserstoff oder $C_1$—$C_6$-Alkyl bedeuten) oder einem Metallkomplex der Formel (4):

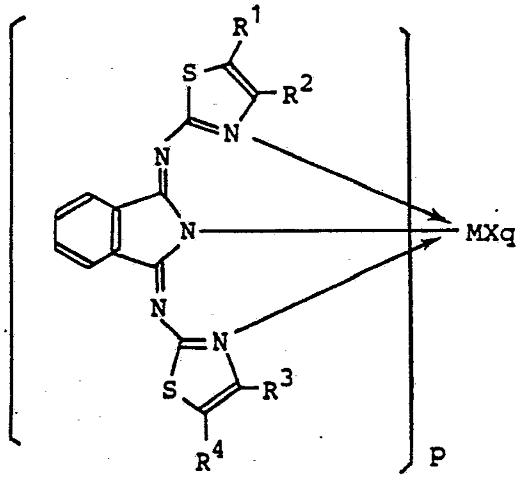

(4)

(worin M Co, Mn, Cr oder Fe bedeutet, X für einen anionischen Liganden steht, $R^1$, $R^2$, $R^3$ und $R^4$, die gleich oder verschieden sein können, je Wasserstoff oder $C_1$—$C_6$-Alkyl bedeuten, P 1 oder 2 bedeutet und q 0, 1 oder 2 bedeutet) als Katalysator durchgeführt wird.

2. Verfahren nach Anspruch 1, dadurch gekennzeichnet, daß das Cycloalkan, welches mit molekularem Sauerstoff oxidiert wird, 5 bis 12 Kohlenstoffatome enthält.

3. Verfahren nach Anspruch 1 oder 2, dadurch gekennzeichnet, daß das Metall M in der Metallverbindung (2) und in dem Metallkomplex (4) Co ist.

4. Verfahren nach Anspruch 1, 2 oder 3, dadurch gekennzeichnet, daß der anionische Ligand X in der Metallverbindung (2) und in dem Metallkomplex (4) eine organische Säuregruppe, eine anorganische Säuregruppe oder Sauerstoff ist.

5. Verfahren nach einem der vorhergehenden Ansprüche, dadurch gekennzeichnet, daß die Konzentration an Cycloalkylhydroperoxid 0,1 bis 10 Gew.-% beträgt.

6. Verfahren nach einem der vorhergehenden Ansprüche, dadurch gekennzeichnet, daß das Molverhältnis von Isoindolin (3) zur Metallverbindung (2) 0,1/1 bis 100/1 beträgt.

7. Verfahren nach einem der vorhergehenden Ansprüche, dadurch gekennzeichnet, daß die Menge an Metallverbindung (2) oder Metallkomplex (4), ausgedrückt als Metall, 0,1 bis 100 ppm, bezogen auf das gesamte Gemisch, beträgt.

8. Verfahren nach einem der vorhergehenden Ansprüche, dadurch gekennzeichnet, daß die Reaktionstemperatur 80 bis 200°C beträgt.

9. Verfahren nach einem der vorhergehenden Ansprüche, dadurch gekennzeichnet, daß die Reaktionszeit 1 bis 120 Minuten beträgt.

10. Verfahren nach einem der vorhergehenden Ansprüche, dadurch gekennzeichnet, daß der Reaktionsdruck 0,2 bis 2 MPa gauge (2 bis 20 kg/$cm^2$ G) beträgt.

**Revendications**

1. Un procédé pour produire un mélange contenant une cycloalcanone et/ou un cycloalcanol, qui comprend l'oxydation d'un cycloalcane par de l'oxygène moléculaire pour donner un hydroperoxyde de cycloalkyle représenté par la formule (1):

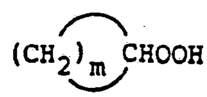

(1)

(avec $4 \leqq m \leqq 11$) et la décomposition de l'hydroperoxyde de cycloalkyle résultant pour produire un mélange contenant une cycloalcanone et/ou un cycloalcanol, caractérisé en ce qu'on accomplit l'oxydation du cycloalcane par de l'oxygène moléculaire et/ou la décomposition de l'hydroperoxyde de cycloalkyle en présence, à titre de catalyseur, soit d'un mélange d'un composé métallique de la formule (2):

$$MX_n \qquad (2)$$

(dans laquelle M représente Co, Mn, Cr ou Fe, X représente un ligand anionique et n est égal à 2 ou 3) et d'une isoindoline de la formule (3):

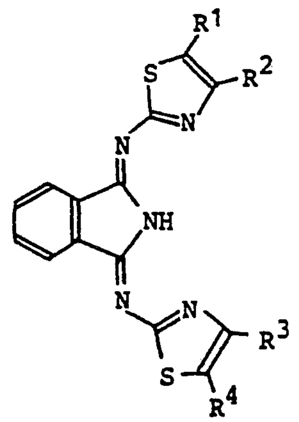

(3)

(dans laquelle $R^1$, $R^2$, $R^3$ et $R^4$, qui peuvent être identiques ou différents, représentent respectivement l'hydrogène ou un alkyle de type $C_1$—$C_6$), soit un complexe métallique représenté par la formule (4):

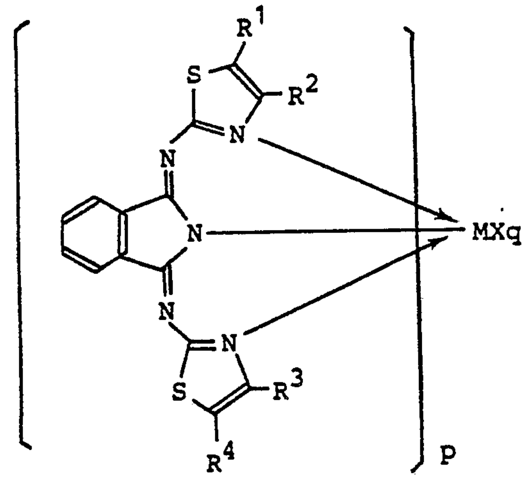

(4)

(dans laquelle M représente Co, Mn, Cr ou Fe, X représente un ligand anionique, $R^1$, $R^2$, $R^3$ et $R^4$, qui peuvent être identiques ou différents, représentent respectivement l'hydrogène ou un alkyle de type $C_1$—$C_6$, p est égal à 1 ou 2 et q est égal à 0, 1 ou 2).

2. Un procédé selon la revendication 1, dans lequel le cycloalcane à oxyder par de l'oxygène moléculaire contient de 5 à 12 atomes de carbone.

3. Un procédé selon la revendication 1 ou 2, dans lequel le métal M dans le composé métallique (2) et le complexe métallique (4) consiste en Co.

4. Un procédé selon la revendication 1, 2 ou 3, dans lequel le ligand anionique X dans le composé métallique (2) et le complexe métallique (4) est un groupe acide organique, un groupe acide inorganique ou de l'oxygène.

5. Un procédé selon l'une quelconque des revendications précédentes dans lequel la concentration de l'hydroperoxyde de cycloalkyle est de 0,1 à 10% en poids.

6. Un procédé selon l'une quelconque des revendications précédentes, dans lequel le rapport molaire de l'isoindoline (3) au composé métallique (2) est de 0,1/1 à 100/1.

7. Un procédé selon l'une quelconque des revendications précédentes, dans lequel la quantité du composé métallique (2) ou du complexe métallique (4), en termes de métal, est de 0,1 à 100 ppm de l'ensemble du mélange.

8. Un procédé selon l'une quelconque des revendications précédentes, dans lequel la température de réaction est de 80 à 200°C.

9. Un procédé selon l'une quelconque des revendications précédentes, dans lequel la durée de réaction est de 1 à 120 minutes.

10. Un procédé selon l'une quelconque des revendications précédentes, dans lequel la pression manométrique de réaction est de 0,2 à 2 MPa (2 à 20 $kg/cm^2$).